# EUROPEAN PATENT APPLICATION

(11) **EP 3 090 752 A1**
(43) Date of publication of application: **09.11.2016**
(21) Application number: 15166746.6
(22) Date of filing: 07.05.2015
(51) Int. Cl.: A61K 35/76, C12N 15/113, C12N 15/86

(54) **ONCOLYTIC VIRUSES WITH AVEN KNOCKDOWN INFORMATION AND THEIR MEDICAL USES**

(71) Applicant: Julius-Maximilians-Universität Würzburg, 97070 Würzburg (DE)
(72) Inventor: Cecil, Alexander, 97072 Würzburg (DE)
(74) Representative: Engelhard, Markus

(57) **Abstract**

The present invention relates to recombinant oncolytic viruses that comprise in their viral genome the genetic information for a si-RNA, sh-RNA, antisense-DNA, antisense-RNA, scAB or sd-AB for inhibiting the gene AVEN in a target or host cell, and pharmaceutical compositions thereof. The present invention further relates to the use of said oncolytic viruses as gene delivery tool and/or for tumor detection. The present invention further relates to the medical uses of said oncolytic viruses, in particular in oncolytic virotherapy and for the diagnosis, prevention and/or treatment of diseases, such as cancer.

SEQUENCE LISTING

<110>  Julius-Maximilians-Universität Würzburg

<120>  Oncolytic viruses with AVEN knockdown information and their
       medical uses

<130>  U30639EP

<160>  10

<170>  PatentIn version 3.5

<210>  1
<211>  25
<212>  DNA
<213>  Artificial Sequence

<220>
<223>  AVEN si-RNA

<400>  1
gccaguguuu acagcauagc agatg                                          25


<210>  2
<211>  25
<212>  DNA
<213>  Artificial Sequence

<220>
<223>  AVEN si-RNA

<400>  2
uguggauagu gaguuauugg uucga                                          25


<210>  3
<211>  25
<212>  DNA
<213>  Artificial Sequence

<220>
<223>  AVEN si-RNA

<400>  3
gcugcugauu aagagaauag augca                                          25


<210>  4
<211>  25
<212>  DNA
<213>  Artificial Sequence

<220>
<223>  control si-RNA

<400>  4
cguuaaucgc guauaauacg cguat                                          25


<210>  5
<211>  20

<212>  DNA
<213>  Artificial Sequence

<220>
<223>  primer

<400>  5
gatttcagtg tcctccttag                                                   20


<210>  6
<211>  20
<212>  DNA
<213>  Artificial Sequence

<220>
<223>  primer

<400>  6
ccttgccatc atcagttctc                                                   20


<210>  7
<211>  20
<212>  DNA
<213>  Artificial Sequence

<220>
<223>  primer

<400>  7
gccttccgtg tccccactgc                                                   20


<210>  8
<211>  20
<212>  DNA
<213>  Artificial Sequence

<220>
<223>  primer

<400>  8
caatgccagc cccagcgtca                                                   20


<210>  9
<211>  20
<212>  DNA
<213>  Artificial Sequence

<220>
<223>  primer

<400>  9
cctctcccaa gtccacacag                                                   20


<210>  10
<211>  16
<212>  DNA
<213>  Artificial Sequence

<220>
<223>  primer

<400>  10
ctgcctccac ccactc                                                    16

## Description

The present invention relates to recombinant oncolytic viruses that comprise in their viral genome the genetic information for a si-RNA, sh-RNA, antisense-DNA, antisense-RNA, sc-AB or sd-AB for inhibiting the gene AVEN in a target or host cell, and pharmaceutical compositions thereof. The present invention further relates to the use of said oncolytic viruses as gene delivery tool and/or for tumor detection. The present invention further relates to the medical uses of said oncolytic viruses, in particular in oncolytic virotherapy and for the diagnosis, prevention and/or treatment of diseases, such as cancer.

### BACKGROUND OF THE INVENTION

An oncolytic virus is a virus that has the potential to infect and destroy cancerous cells (Bartlett et al., 2013; Patil et al., 2012). As the infected cancer cells are destroyed by lysis, they release new infectious virus particles to help destroy the remaining tumor. Oncolytic viruses are thought not only to cause direct destruction of the tumor cells, but also to stimulate host anti-tumour immune responses. However, these viruses are not effective against all types of cancer due to certain resistance mechanisms against oncolytic virus mediated apoptosis (Cecil et al., 2014).

Apoptosis as a mechanism of cell death is essential for tissue homeostasis and developmental processes. Aberrations of the tightly controlled process of apoptosis result in a number of human disorders including cancer, neurodegenerative diseases or autoimmune diseases (Hajra, 2004). Described as one of the hallmarks of cancer, the avoidance of apoptosis to prevent cell death is one crucial step in tumor development (Hanahan *et al.,* 2000; Hanahan 2011). There are two major apoptotoc pathways leading to the activation of the caspase cascade: the extrinsic pathway, activated by extracellular signals via cell death receptors and the intrinsic / mitochondrial pathway activated by endogenous signals such as DNA damage (see e.g. Ledgerwood and Morrison, 2009). The apoptosome complex is a central part of the intrinsic pathway. Its formation is the result of a Bcl-2 family-regulated release of cytochrome C from the mitochondria into the cytosol that binds to the apoptosis protease activating factor 1 (APAF-1). A conformational change of APAF-1 results in the binding of the initiator procaspase-9 (Brenner and Mak, 2009). The apoptosome complex is tightly controlled by a number of endogenous regulator (Bratton and Salvesen, 2010). One of those anti-apoptotic factors is AVEN, recently identified by Chau *et al.* (2010). AVEN is an ubiquitous protein, found to be expressed in a great number of adult tissues and cell lines (Chau *et al.,* 2010; Hawley *et al.,* 2012). It binds to Bcl-xL strengthening its anti-apoptotic activity and also interacts with APAF-1 suppressing the APAF-1 mediated activation of caspase-9 (Chau *et al.,* 2010). In addition, AVEN directly binds and activates the cell-cycle regulating ataxia-telangiectasia (ATM) protein kinase, a critical regulator of the G2/M DNA damage checkpoint in the DNA damage response pathway (Guo *et al.,* 2008). Melzer *et al.* (2012) showed that AVEN is proteolytically cleaved by cathepsin D resulting in the removal of an inhibitory N-terminal domain and the activation of the anti-apoptotic function of the protein. Also, the WNT signaling pathway, and especially the regulation of the glycogen synthase kinase 3 beta (GSK3β), the primary inhibitor of β-Catenin, has been shown to be of great importance to tumor proliferation (Kotliarova *et al.,* 2008) β-Catenin has been reported previously to be responsible for tumor proliferation, e.g. in basal cell carcinoma (Saldanha *et al.,* 2004) or in prostate cancer (Kypta and Waxman, 2012).

The specific interference and modifications of molecules and factors being part of the apoptotic cell death pathways such as the apoptosome complex are of special interest in the development of novel cancer therapeutics (see e.g. Ledgerwood and Morrison, 2009). Oncolytic virotherapy is one such promising method (see e.g. the review Goldufsky *et al.,* 2013). It is defined by activation of apoptosis and/or necrosis in tumor cells leading to the specific destruction and lysis of neoplastic tissues, all the while normal tissues remain unharmed (see e.g. Bourke *et al.,* 2011; Liu *et al.,* 2007; Chen and Szalay, 2011). Oncolytic vaccinia viruses (VACVs) have been demonstrated to be very promising candidates to combat different tumor types in several pre-clinical and clinical trials (see e.g. Advani *et al.,* 2012; Donat *et al.,* 2012; Donat *et al.,* 2014; Ehrig *et al.,* 2013; Weibel *et al.,* 2013-a; Zhang *et al.,* 2007). Some tumor cell lines, however, show only a weak response to oncolytic VACV therapy compared to other cell lines (Worschech *et al.,* 2009).

Thus, there is a need in the art for improved means and methods for oncolytic virotherapy as well as for improved oncolytic viruses.

### SUMMARY OF THE INVENTION

According to the present invention this object is solved by a recombinant virus comprising or carrying in its viral genome the genetic information for inhibiting the gene AVEN in a target or host cell, wherein said genetic information is selected from genetic information for a si-RNA, sh-RNA, antisense-DNA, antisense-RNA, sc-AB or sd-AB.

According to the present invention this object is solved by a pharmaceutical composition, comprising
(i) the recombinant virus of the present invention; and
(ii) optionally, pharmaceutically acceptable carrier(s) and/or excipient(s).

According to the present invention this object is solved by using the the recombinant virus of the present invention or the pharmaceutical composition of the present invention, as gene delivery tool and/or for tumor detection.

According to the present invention this object is solved by providing the recombinant virus of the present invention or the pharmaceutical composition of the present invention for use in medicine.

According to the present invention this object is solved by providing the recombinant virus of the present invention or the pharmaceutical composition of the present invention for use in the diagnosis, prevention and/or treatment of a disease, wherein said disease is cancer.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "20 to 100 nucleotides" should be interpreted to include not only the explicitly recited values of 20 to 100, but also include individual values and sub-ranges within the indicated range. Thus, included in this numerical range are individual values such as 20, 21, 22, 23, 24, 25, 26, 27, 28, 29 ,... 97, 98, 100 and sub-ranges such as from 25 to 35, from 20 to 40, from 25 to 50, etc. This same principle applies to ranges reciting only one numerical value, such as "at least 25 nucleotides". Furthermore, such an interpretation should apply regardless of the breadth of the range or the characteristics being described.

### Oncolytic viruses for AVEN knockdown

As discussed above, the present invention provides recombinant viruses.

The recombinant viruses comprise or carry in their viral genome the genetic information for inhibiting the gene AVEN in a target or host cell, in particular in a target or host cell infected by the virus.

According to the invention, the AVEN gene in an infected target or host cell is to be inhibited by gene silencing or gene knockdown.

The terms "gene silencing" or "gene knockdown" as used herein refer to the ability to reduce or prevent the expression of a certain gene in a target or host cell. Gene silencing can occur during either transcription or translation.

According to the invention, said genetic information, which is comprised or carried in the recombinant viruses of the invention, for inhibiting the gene AVEN is selected from genetic information for a
silencing RNA (si-RNA),
small hairpin RNA (sh-RNA),
antisense-DNA,
antisense-RNA,
single chain antibody (sc-AB), or
single domain antibody (sd-AB).

Preferably, the recombinant viruses of the present invention are selected from:
- the *Poxviridae, Adenovinidae, Reoviridae* and *Herpesviridae* families of double stranded DNA (dsDNA) viruses,
- the *Picornaviridae, Rhabdoviridae, Panamyxovinidae* and *Togaviridae* families of single stranded RNA (ssRNA) viruses, and
- the *Retroviridae* family of retro-transcribing viruses.

An "oncolytic virus" as used herein refers to a virus that has the potential to infect and destroy target or host cells, in particular cancerous cells.

The oncolytic virus is therefore selected from the *Poxviridae, Adenoviridae, Reoviridae* and *Herpesviridae* families of double stranded DNA viruses, or from the *Picornaviridae, Rhabdoviridae, Paramyxoviridae* and *Togaviridae* families of single stranded RNA viruses, or from the *Retroviridae* family of retro-transcribing viruses. Their viral genomes are to be modified to carry the genetic information for expression of a si-RNA, sh-RNA, antisense-DNA, antisense-RNA, sc-AB or sd-AB for inhibiting the gene AVEN in an infected target or host cell.

In one embodiment, the recombinant virus, in particular the recombinant oncolytic virus is
- an attenuated oncolytic virus,
   such as
   an attenuated adenovirus (e.g. ONYX-105),
   an attenuated vaccinia virus (VACV),
   an attenuated poliovirus (e.g. PV1),
- a replication-competent oncolytic virus,
   such as
   a replication-competent oncolytic adenovirus (e.g. ONYX-411),
   a replication-competent oncolytic vaccinia virus (VACV),
   a replication-competent oncolytic seneca valley virus (e.g. SVV-01),
   a replication-competent oncolytic reovirus.

In one embodiment, the genetic information for said AVEN si-RNA, sh-RNA, antisense-DNA, antisense-RNA, sc-AB or sd-AB is combined with / linked to a late/very late promoter.

In one embodiment, said late/very late promoter is selected from:
- PE/L (early/late promoter), or
- P11 (late/very late promoter).

Preferebly, the recombinant oncolytic virus comprises or carries
- in its viral genome deletional mutation(s) in nonessential gene(s) and/or in small ORF(s),
- foreign or heterologous genes,
   such as
   - genes encoding for tumor suppressor proteins, immunostimulatory proteins and cytokines,
   - genes encoding for pro-drug activating enzymes,
   - genes encoding for marker proteins,
      such as fluorescent proteins, e.g. GFP or its derivatives,
   - genes encoding for glucoronidase as a marker for oncolysis.

In one embodiment, the recombinant oncolytic virus is an oncolytic vaccinia virus (VACV) which comprises or carries in its viral genome deletional mutation(s)
- in nonessential gene(s),
   such as *JR2* (encoding thymidine kinase, TK), *A56R* (encoding hemagglutinin), vaccinina growth factor gene,
   and/or
- in small ORF(s)
   such as *F14.5,*
   and comprises or carries foreign or heterologous genes,
   such as
   renilla luciferase-aequorea GFP (Ruc-GFP), and/or
   glucoronidase as a marker for oncolysis, and/or
   transferrin receptor (TFR) and *lacZ.*

In one embodiment, the recombinant oncolytic virus is for example the oncolytic vaccinia virus strain GLV-1h68.

In one embodiment, the si-RNA for AVEN comprises a nucleic acid sequence which comprises preferably about 20 to about 100 nucleotides, more preferably at least 25 nucleotides, of which preferably at least 23 are ribonucleotides and at least two are desoxyribonucleotides.

In one embodiment, the si-RNA for AVEN comprises or consists of the nucleic acid sequence of any one of SEQ ID NOs. 1 to 3:

In one embodiment, the si-RNA for AVEN comprises or consists of the nucleic acid sequence of SEQ ID NO. 1:
SR311345A rGrCrCrArGrUrGrUrUrUrArCrArGrCrArUrArGrCrArGrATG Wherein "r" denotes the sequence being RNA and not DNA.

In one embodiment, the si-RNA for AVEN comprises or consists of the nucleic acid sequence of SEQ ID NO. 2:
SR311345B rUrGrUrGrGrArUrArGrUrGrArGrUrUrArUrUrGrGrUrUrCGA Wherein "r" denotes the sequence being RNA and not DNA.

In one embodiment, the si-RNA for AVEN comprises or consists of the nucleic acid sequence of SEQ ID NO. 3:
SR311345C rGrCrUrGrCrUrGrArUrUrArArGrArGrArArUrArGrArUrGCA Wherein "r" denotes the sequence being RNA and not DNA.

Preferably, the target or host cell is a cancerous or tumor cell, which is to be infected by the oncolytic virus of the invention.

### Pharmaceutical compositions

As discussed above, the present invention provides pharmaceutical compositions, comprising
(i) the recombinant (oncolytic) virus of the present invention; and
(ii) optionally, pharmaceutically acceptable carrier(s) and/or excipient(s).

In one embodiment, the pharmaceutical composition of the present invention comprises further drug(s), such as
- chemotherapeutic agent(s),
- radiotherapeutic agent(s), and/or
- agent(s) for near infrared laser-induced targeted cancer therapy.

In one embodiment, the pharmaceutical composition of the present invention is formulated for systemic delivery, tumor injection, intravenous administration, and/or for intradermal, subcutaneous, intramuscular, intravenous, intraosseous, intraperitoneal, intrathecal, epidural, intracardiac, intraarticular, intracavernous, intracerebral, intracerebroventricular and intravitreal injection(s).

### Medical uses

As discussed above, the present invention provides the use of the recombinant (oncolytic) virus of the present invention or the pharmaceutical composition of the present invention, as gene delivery tool and/or for tumor detection.

As discussed above, the present invention provides the recombinant (oncolytic) virus of the present invention or the pharmaceutical composition of the present invention, for use in medicine.

As discussed above, the present invention provides the recombinant (oncolytic) virus of the present invention or the pharmaceutical composition of the present invention, for use in the diagnosis, prevention and/or treatment of a disease.

Said disease is preferably cancer.

In one embodiment, the recombinant oncolytic virus or pharmaceutical composition is for use in oncolytic virotherapy.

The term "oncolytic virotherapy" as used herein refers to therapy of cancer by administration of oncolytic viruses to induce tumor regression.

In one embodiment, the recombinant oncolytic virus or pharmaceutical composition is for use in combination with other therapies, preferably other cancer therapies, such as chemotherapy and/or radiation therapy.

Preferably, the administration is systemic, intravenous, via injection into tumor, and/or via intradermal, subcutaneous, intramuscular, intravenous, intraosseous, intraperitoneal, intrathecal, epidural, intracardiac, intraarticular, intracavernous, intracerebral, intracerebroventricular and intravitreal injection(s).

### Methods of treatment

As discussed above, the present invention provides a method for the diagnosis, prevention and/or treatment of a disease,
comprising the step of
administering a therapeutically effective amount of a recombinant (oncolytic) virus of the present invention or a pharmaceutical composition of the present invention to a subject in need thereof.

Said disease is preferably cancer.

A therapeutically effective amount of a recombinant (oncolytic) virus of the present invention is the amount which results in the desired therapeutic result, in particular tumor regression.

The recombinant viruses are preferably administered in multiple cycles over a period of time, such as for several days up to several weeks.

For example, a therapeutically effective amount of VACV can be in the range from about 10⁵ pfu and about 10⁹ pfu, which is preferably delivered to the subject/patient in need thereof in multiple cycles, such as via a 3 cycle regimen over about 28 days.

The skilled artisan will be able to determine suitable therapeutically effective amounts.

As discussed above, the recombinant oncolytic virus or pharmaceutical composition can be administered in combination with other therapies, preferably other cancer therapies, such as chemotherapy, radiation therapy and/or near infrared laser-induced targeted cancer therapy.

As discussed above, the administration is systemic, intravenous, via injection into tumor, and/or via intradermal, subcutaneous, intramuscular, intravenous, intraosseous, intraperitoneal, intrathecal, epidural, intracardiac, intraarticular, intracavernous, intracerebral, intracerebroventricular and intravitreal injection(s).

In one embodiment, the method comprises oncolytic virotherapy.

### Further description of preferred embodiments

Oncolytic viruses have the potential to infect and destroy cancerous cells (Bartlett et *al.,* 2013; Patil et al., 2012). However, these viruses are not effective against all types of cancer due to certain resistance mechanisms against oncolytic virus mediated apoptosis (Cecil et al., 2014). One of the most crucial resistance mechanisms is found in the suppression of the apoptosome. The apoptosome complex is a central part of the intrinsic pathway. Its formation is the result of a Bcl-2 family-regulated release of cytochrome C from the mitochondria into the cytosol that binds to the apoptosis protease activating factor 1 (APAF-1). A conformational change of APAF-1 results in the binding of the initiator procaspase-9 (Brenner and Mak, 2009; Bao 2007). The apoptosome complex is tightly controlled by a number of endogenous regulators (Bratton and Salvesen, 2010).

One of those anti-apoptotic factors is AVEN, recently identified by Chau *et al.* (2000). AVEN is a ubiquitous protein, found to be expressed in a great number of adult tissues and cell lines (Chau *et al.,* 2000; Hawley *et al.,* 2012). It binds to Bcl-xL and thus strengthening its anti-apoptotic activity (Figueroa *et al.,* 2004). It also interacts with APAF-1: it suppresses the APAF-1 mediated activation of caspase-9 (Chau *et al.,* 2000).

In a clinical study, Paydas *et al.* (2003) found elevated AVEN gene expression levels in adult acute leukemia patients: Higher levels were found in those patients with relapse compared to those without relapse. Also in childhood acute lymphoblastic leukemia, poor prognosis was associated with AVEN overexpression (Choi *et al.,* 2006). Eißmann *et al.* (2012) concluded in their study an oncogenic potential of AVEN during development of hematopoietic neoplasms. A microarray study revealed decreased nuclear AVEN gene expression levels in breast cancer tissue compared to healthy breast tissue - providing evidence that expression of AVEN leads to resistance against apoptosis in breast cancer tissue induced by DNA damage (Kutuk et al., 2010).

By genetically modifying the genome of a suitable oncolytic virus - as for example GLV-1h68 - it is possible to perform a knockdown of the expression of AVEN and thus availability of this anti-apoptotic factor inside the tumor cells. To this end the genetic code for small interfering RNA targeting the gene AVEN (si-AVEN) is put onto the genome of an oncolytic virus and preferably be coupled with a late/very late promotor. This leads to the expression of si-AVEN after replication of the virus has taken place in the infected tumor cell. The knockdown of AVEN subsequently leads to enhanced apoptosis of cancer strain cells, when oncolytic virus therapy is performed.

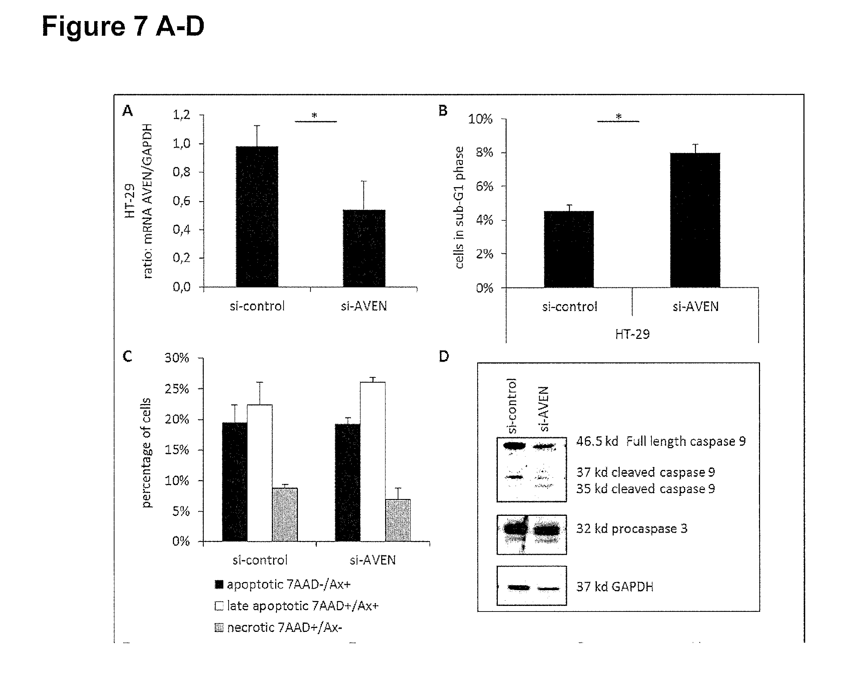

To provide proof of concept, human colorectal adenocarcinoma cells (HT-29) were transfected for 48 to 72 h with si-AVEN and a scrambled RNA (si-control) as control. AVEN gene expression was significantly down-regulated in HT-29 cells compared to cells transfected with si-control (shown in Figure 7A). To verify whether the knockdown of AVEN would enhance apoptotic characteristics in the cancer cells, DNA fragmentation as one characteristic to distinguish apoptotic from necrotic cell death (Gentschev *et al.,* 2010) was tested. Down-regulation of the anti-apoptotic factor AVEN in HT-29 cells revealed a significant increase of DNA-fragmentation, detected as an increase of cells in sub-G1 phase in si-AVEN transfected cells compared to si-control transfected cells (shown in Figure 7B). No significant difference could be observed in Annexin⁺/7AAD⁺/⁻ (apoptotic) or Annexin⁻/7AAD⁺ (necrotic) cells between si-control and si-AVEN transfected HT-29 cells (shown in Figure 7C). Western Blot analysis revealed the expression of procaspase-3 (32 kda in both si-AVEN and si-control transfected cells. The cleaved and activated form of the effector caspase-3 was not expressed (shown in Figure 7D). In accordance with the literature, down-regulation of AVEN results in an activation and cleavage of the initiator caspase-9 (35 kd) (shown in Figure 7D). GAPDH was used as loading control.

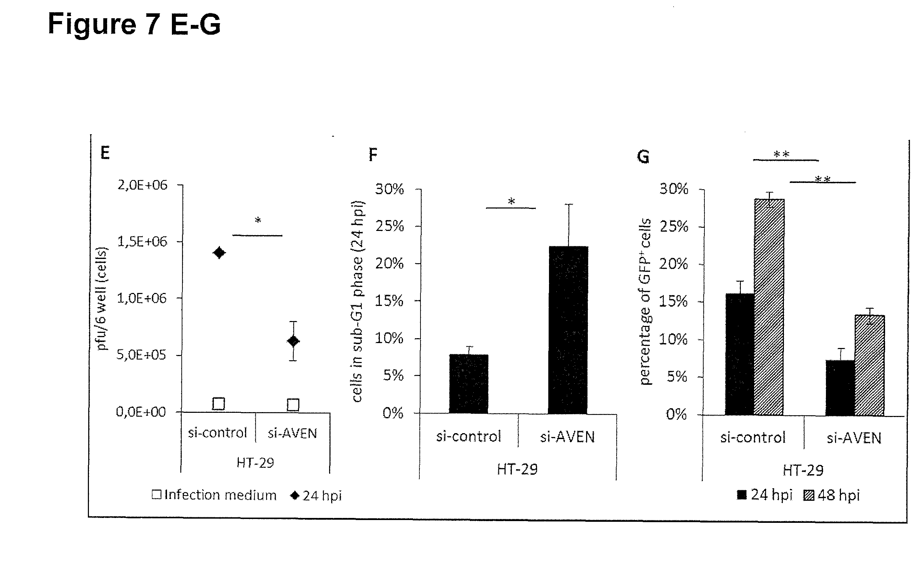

Following the functional proof of AVEN down-regulation in HT-29 cells, replication of one oncolytic virus from the Genelux corporation (GLV-1h68) was tested at a MOI of 1.0 and the infection rate (GFP⁺ cells) 24 and 48 hpi in these cells. A significant reduction of the virus titer (shown in Figure 7E) and a significantly smaller number of GFP⁺ cells (shown in Figure 7F) were detected in si-AVEN transfected cells compared to si-control transfected cells at 24 and 48 hpi. Percentage of cells in sub-G1 phase 24 hpi was significantly enhanced in GLV-1h68 infected si-AVEN transfected HT-29 cells compared to si-control transfected HT-29 cells (shown in Figure 7G). Taken together, a reduced infection rate (GFP⁺ cells) and an impaired viral replication took place after down-regulation of the anti-apoptotic factor AVEN in HT-29 cells. However, this down-regulation also resulted in an increase of apoptosis in HT-29.

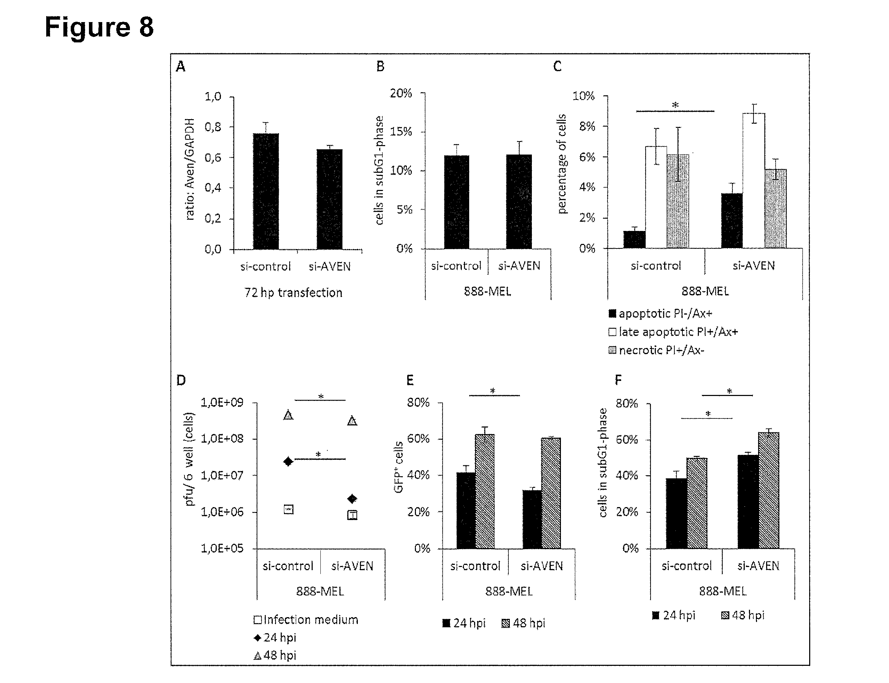

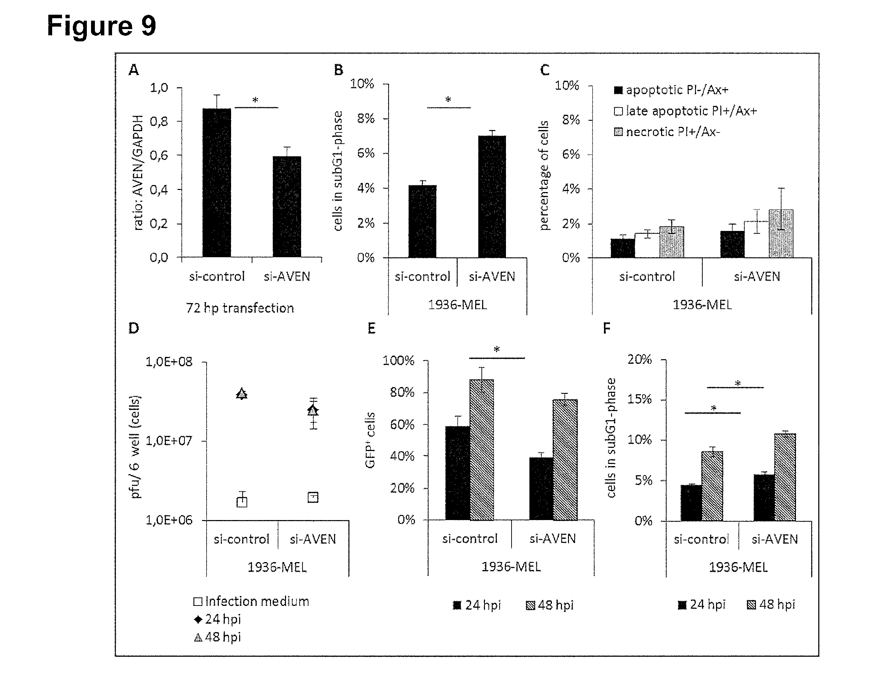

To see, whether the observed oncolytic virus effects are a general phenomenon or unique for the cell line HT-29, knockdown of AVEN was also tested in metastatic melanoma cancer cell lines 888-MEL (see Figures 8A-F) and 1936-MEL (see Figures 9A-F). In these tests similar results could be obtained: viral replication is reduced but at the same time apoptosis of cancer strains is enhanced.

This novel approach can also be adapted to various different oncolytic viruses: the genetic layout of a wide array of these viruses is very similar and easily modifiable (Table 1). These viruses have similar characteristics regarding tumor regression (Chen *et al.,* 2011), which can be vastly improved by the invention.

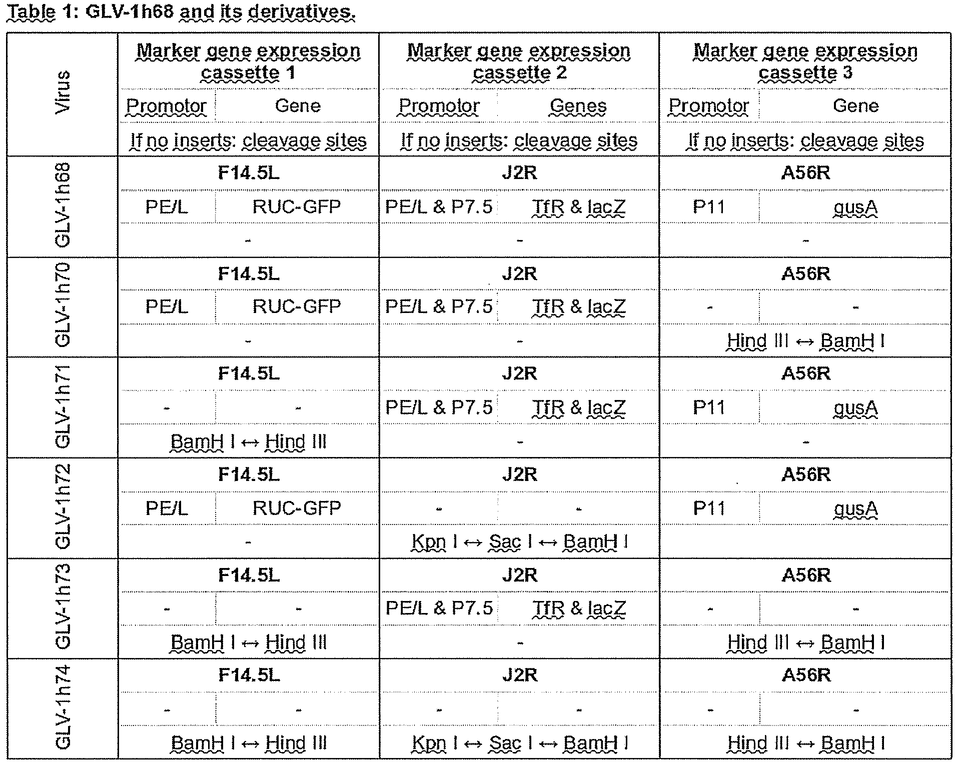

### - Abstract

Vaccinia Virus GLV-1h68 has been proven to be a viable candidate for cancer treatment and is currently being tested in several clinical trials. However, cell culture studies revealed that GLV-1h68 is less effective against certain kinds of tumor cell lines such as the human colon adenocarcinoma cell line HT-29 or the human melanoma cell line 1936-MEL whereas it is very effective against other cell lines such as 888-MEL another human melanoma cell line. In this study we calculate the effects of the genetic variances in four different cell lines (HT-29, GI-101A, 1936-MEL, and 888-MEL) *in silico* by a dynamic modeling approach and by analytical testing in cell culture. We also establish a method of RNA interference to render the non- or weak-responding tumor cell lines susceptible to GLV-1h68. With these methods we found a very strong correlation of deactivation of the gene AVEN, an anti-apoptotic effector, and susceptibility to GLV-1h68. By deactivation of AVEN we were able to restore the full efficacy of GLV-1h68 and only marginally decrease its tumor infection rate.

In order to understand why some tumor cells are able to survive an infection by the oncolytic VACV GLV-1h68, we statistically evaluated the gene expression data from infected and non-infected melanoma cell lines (888-MEL, 1936-MEL), the breast cancer cell line GI-101A, as well as colon adenocarcinoma cells HT-29. For convenience, the evaluated gene expression data acquired were visualized by heatmapping. Boolean modeling of the apoptosis network was also performed and the significant gene expression changes were mapped onto the models. Based on this data, a special focus was set on the expression and function of the anti-apoptotic protein AVEN in different cancer cell lines. We elucidated that there is a specific role and function of AVEN for successful infection and replication of GLV-1h68 in those tumor cells and that therapeutic success or failure is dependent on AVEN levels in the cancer strains.

### - Conclusion

Boolean modeling in combination with gene expression analysis proved viable to identify a target gene responsible for reduced apoptosis in HT-29 tumor cells. This gene, not found to be significantly up-regulated in GI-101A, suppresses the effects of GLV-1h68 in HT-29. The Boolean models also predicted the effects of a si-RNA knockdown of AVEN: the efficacy of GLV-1h68 against HT-29 could be restored. *In vitro* the effects of this si-RNA knockdown were not as expected by Boolean modeling. The rate of infection with GLV-1h68 was reduced, however the rate of apoptosis induced by GLV-1h68 also increased significantly.

We also found *AVEN in vitro* in 888-MEL and 1936-MEL cells. AVEN does also not show up to be significantly up-regulated in the gene expression data of those two cell lines we evaluated. The effects of AVEN in 888-MEL and 1936-MEL, due to their non-significant expression compared to HT-29, *show in vitro* to be not strong enough to impede GLV-1h68 efficacy as they do in HT-29. However, 1936-MEL does not react as strongly to GLV-1h68 as 888-MEL does. A subsequent si-RNA knockdown of AVEN in 1936-MEL improves viral efficacy in the weak responder 1936-MEL. These findings are in accordance with the Boolean models for 888-MEL and 1936-MEL. However, according to Boolean modeling, in the melanoma cancer strains the effects of casein kinases and their role to regulate GSK3β seem to be more predominant then the effect of AVEN.

Therefore, the expression of AVEN and up-regulation of casein kinases could also serve as biomarkers for tumor proliferation.

Moreover, by identification of AVEN as one of the most important regulators of resistance against a treatment of cancer by oncolytic virus therapy, we herewith describe new designs for more specific viruses against cancer strains with actively expressed AVEN. By inserting a si-RNA for AVEN on a late/very late promoter into the viral genome, we are now able to provide effectively treatment of various cancer strains in humans. This method is also applicable for sh-RNA, antisense-DNA, antisense-RNA, sc-AB or sd-AB for gene knockdown of AVEN.

The following examples and drawings illustrate the present invention without, however, limiting the same thereto.

### BRIEF DESCRIPTION OF THE DRAWINGS

**Figure 1****.** *Genes from the human melanoma cell line 1936-MEL associated to the apoptosis signaling network with the highest activities overall.*
   Especially the casein kinases are of interest, as they are significantly upregulated. This leads to a higher inhibition of GSK3β and subsequently higher activation of β-Catenin. β-Catenin in turn is then responsible for uncontrolled tumor proliferation.
**Figure 2****.** *Upregulation of the genes associated to the apoptosis network for the Human melanoma cell line 888-MEL.*
   As for 1936-MEL, the casein kinases are of special interest due to their influence on β-Catenin through the WNT- and Hedgehog signaling cascades and its subsequent effect of uncontrolled tumor proliferation.
**Figure 3****.** *Differences in gene expression of HT-29 and GI-101A cancer strains.*
   Especially interesting is the difference in expression of AVEN, which is highly upregulated only in HT-29.
**Figure 4****.** *Characterization of GLV-1h68 permissiveness in various human tumor cell lines.*
   (A) MTT assay was performed to detect the percentage of living cells (888-MEL, 1936-MEL, GI101A and HT-29) after infection with GLV-1h68 (MOI 0.01, 0.1 and 1.0).
   **(B)** Viral replication in 888-MEL, 1936-MEL, GI-101A, HT-29 cells, infected with GLV-1h68 at an MOI of 0.01 was analyzed by standard viral plaque assay in triplicate. The red line separates active replication from no replication efficiency.

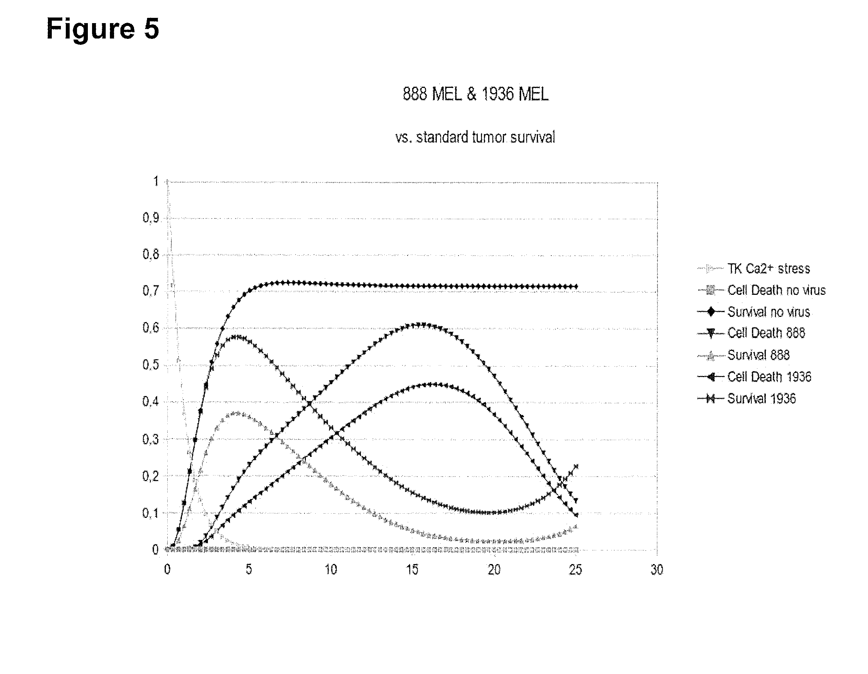

**Figure 5****.** *Boolean calculations of survival vs. apoptosis ratios of different cancer strains.* 1936-MEL shows a twice as high survival rate as 888-MEL when compared to *an in silico* untreated mock cancer strain, whereas the rate of cell death is higher in 888-MEL as in 1936-MEL. Therefore these differences in gene expression are enough to render the GLV-1h68 against 1936-MEL almost ineffective, whereas it is very effective against 888-MEL.

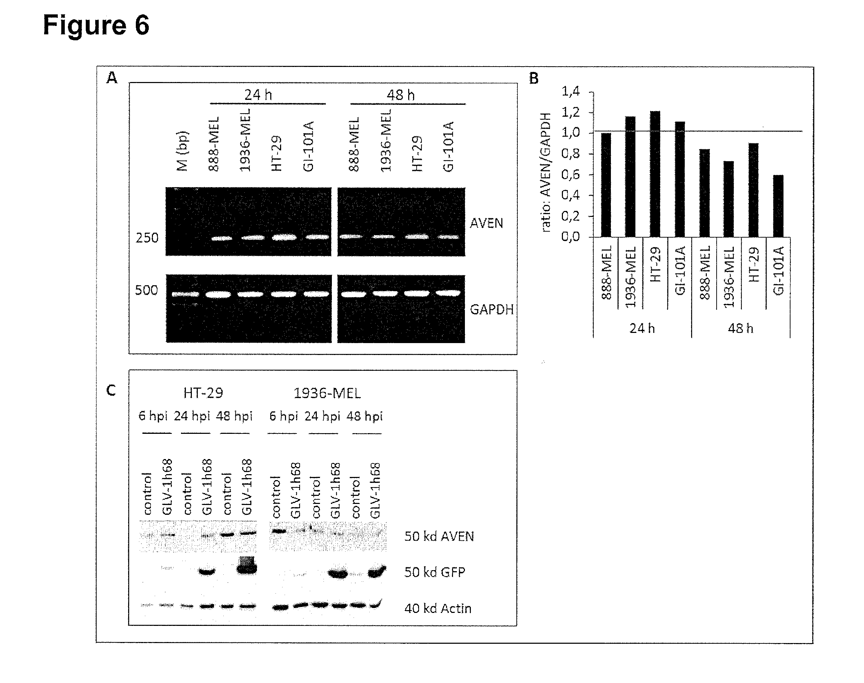

**Figure 6****.** *Expression levels of AVEN in human tumor cell lines.*
   (**A**) *AVEN* gene expression levels in 888-MEL, 1936-MEL, GI-101A and HT-29 cultured for 24 or 48 h were analyzed by PCR. PCR reactions were performed on templates of cDNA from these human cancer cell lines using a set of primers for *AVEN* and *GAPDH* as internal control.
   **(B)** The bar chart represents the ratio between *AVEN* gene expression and *GAPDH* expression in the analyzed cell lines at 24 and 48 hours. The red line marks a ratio of 1.0.
   **(C)** The protein expression of AVEN and GAPDH in HT-29 and 1936-MEL cells infected with GLV-1h68 (MOI 1.0) at 6, 24 and 48 hpi. Mock-infected cells were used as control. GFP expression represents viral infection. The experiments were performed 2-3 times.
**Figure 7****.** *AVEN interference enhances apoptotic characteristics and inhibits GLV-1h68 replication in HT-29 cells.*
   **(A)** Downregulation of *AVEN* mRNA expression in HT-29 cells transfected with AVEN si-RNA (si-AVEN) or scrambled control RNA (si-control). Cells were transfected for 72 h.
   **(B)** The percentage of si-control or si-AVEN transfected HT-29 cells in sub-G1 phase indicating DNA fragmentation (apoptotic characteristic) measured by FACS analysis.
   **(C)** For analyzing cell membrane permeability, transfected cells were stained with the vital dye 7AAD and Annexin (Ax) and were subsequently analyzed by FACS analysis.
   **(D)** Protein expression levels of caspase-9 and caspase-3 were analyzed in si-control and si-AVEN transfected HT-29 cells by western blot analysis. GAPDH was used as loading control.
   **(E)** Viral replication of GLV-1h68 (MOI 1.0) was analyzed in si-control and si-AVEN transfected HT-29 cells by standard viral plaque assay 24 hpi.
   **(F)** Percentage of transfected and infected HT-29 cells in sub-G1 phase were analyzed by FACS analysis.
   **(G)** In parallel to the virus titer, the infection rate of the samples was determined by measuring the percentage of GFP⁺-cells by FACS analysis 24 and 48 hpi.
All transfection experiments were performed in 6-well plates in triplicate and repeated in an independent experiment. Two-sided t-test with unequal variances was used for statistics *p<0.05, ** p< 0.01, ***p<0.001.
**Figure 8****.** *Knockdown of AVEN results in impaired GLV-1h68 replication in 888-MEL cells.*
   **(A)** Downregulation of *AVEN* mRNA expression in 888-MEL cells transfected with AVEN si-RNA (si-AVEN) or scrambled control RNA (si-control). Cells were transfected for 72 h.
   **(B)** The percentage of si-control or si-AVEN transfected 888-MEL cells in sub-G1 phase indicating DNA fragmentation (apoptotic characteristic) measured by FACS analysis.
   **(C)** For analyzing cell membrane permeability, transfected cells were stained with PI and Annexin (Ax) and were subsequently analyzed by FACS analysis.
   **(D)** Viral replication of GLV-1h68 (MOI 0.5) was analyzed in si-control and si-AVEN transfected 888-MEL cells by standard viral plaque assay 24 and 48 hpi.
   **(E)** In parallel to the virus titer, the infection rate of the samples was determined by measuring the percentage of GFP⁺-cells by FACS analysis 24 and 48 hpi.
   **(F)** Percentage of transfected and infected 888-MEL cells in sub-G1 phase 24 hpi and 48 hpi were analyzed by FACS analysis.
   All transfection experiments were performed in 6 well plates in triplicate and repeated two times. Two-sided t-test with unequal variances was used for statistics *p<0.05, ** p< 0.01, ***p<0.001.
**Figure 9****.** *Knockdown of AVEN results in impaired GLV-1h68 replication in 1936-MEL cells.*
   (**A**) Downregulation of AVEN mRNA expression in 1936-MEL cells transfected with AVEN si-RNA (si-AVEN) or scrambled control RNA (si-control). Cells were transfected for 72 h.
   **(B)** The percentage of si-control or si-AVEN transfected 1936-MEL cells in sub-G1 phase indicating DNA fragmentation (apoptotic characteristic) measured by FACS analysis.
   **(C)** For analyzing cell membrane permeability, transfected cells were stained with PI and Annexin (Ax) and were subsequently analyzed by FACS analysis.
   **(D)** Viral replication of GLV-1h68 (MOI 0.5) was analyzed in si-control and si-AVEN transfected 1936-MEL cells by standard viral plaque assay 24 and 48 hpi.
   **(E)** In parallel to the virus titer, the infection rate of the samples was determined by measuring the percentage of GFP⁺-cells by FACS analysis 24 and 48 hpi.
   **(F)** Percentage of transfected and infected 1936-MEL cells in sub-G1 phase 24 hpi and 48 hpi were analyzed by FACS analysis.
      All transfection experiments were performed in 6 well plates in triplicate and repeated two times. Two-sided t-test with unequal variances was used for statistics *p<0.05, ** p< 0.01, ***p<0.001.

### EXAMPLES

### 1. Material and Methods

### 1.1 Virus

The generation of the oncolytic vaccinia virus strain GLV-1h68 was described in detail by Zhang *et.al.* (2007). In brief, GLV-1h68 is a genetically modified attenuated virus construct based on the LIVP strain genome that consists of three expression cassettes namely *Renilla* luciferase-green fluorescent protein (RUC-GFP) fusion protein, β-galactosidase and β-glucuronidase that were recombined into the F14.5L, J2R (encoding thymidine kinase) and A56R (enoding hemagglutinin) loci of the viral genome.

### 1.2 Cell culture

African green monkey kidney fibroblasts (CV-1 cells) were purchased from the American Type Culture Collection (ATCC) and cultured in DMEM (Sigma-Aldrich, Steinheim, Germany) supplemented with 10% fetal bovine serum (FBS; PAA Laboratories, Cölbe, Germany) and penicillin G/streptomycin solution (100 U/ml; Sigma-Aldrich). The human colorectal adenocarcinoma cell line HT-29 (Duke's type B) was obtained from ATCC. Cells were cultured in RPMI-1640 (Sigma-Aldrich) supplemented with 10% FBS, penicillin G/streptomycin solution (100 U/ml) and sodium bicarbonate solution 7.5% (2.2 g/l; Sigma-Aldrich). The human melanoma cell lines 1936-MEL and 888-MEL were kindly provided by F. M. Marincola (National Institutes of Health, Bethesda, MD, USA) (Ascierto *et al.,* 2011; Reinboth *et al.,* 2012; Weibel *et al.,* 2013b) and cultured in RPMI-1640 supplemented with 10% FBS and penicillin G/streptomycin solution (100 U/ml). The human ductal breast adenocarcinoma cell line GI-101A was kindly provided by A. Aller (Rumbaugh-Goodwin Institute for Cancer Research, Inc., FL, USA)) (Ascierto *et al.,* 2011; Weibel *et al.,* 2011) and maintained in RPMI-1640 supplemented with 5 ng/ml b-estradiol (Sigma-Aldrich) and 5 ng/ml progesterone (Sigma Aldrich), 1 mM sodium pyruvate (Sigma-Aldrich), 10 mM HEPES (PAA Laboratories), 20% FBS and penicillin G/streptomycin solution (100 U/ml). Cells were maintained at 37°C and 5% CO₂.

### 1.3 Reverse Transcription (RT)-PCR

For total RNA isolation the RNeasy Mini Kit (Qiagen GmbH, Hilden, Germany) was used according to the manufacturer's instructions. Genomic DNA was excluded from the samples using DNA-*free*™ Kit (Ambion, Austin, TX, USA) according to the manufacturer's instructions. The amount of RNA was measured with a Nanodrop and RNA was converted to cDNA using the RevertAid™ First Strand cDNA Synthesis Kit (Fermentas, St. Leon-Rot, Germany) or the Transcriptor High Fidelity cDNA Synthesis Kit (Roche Diagnostics, Mannheim, Germany). Sequences for human AVEN, human β-Actin and human GAPDH on mRNA level were amplified with Phusion DNA Polymerase (Finnzymes, Espoo, Finland) by PCR with specific primers for
- human AVEN
   forward 5'-GATTTCAGTGTCCTCCTTAG-3' SEQ ID NO. 5, and
   reverse 5'-CCTTGCCATCATCAGTTCTC-3' SEQ ID NO. 6, (according to Paydas, 2003);
- human GAPDH
   forward 5'-GCCTTCCGTGTCCCCACTGC-3' SEQ ID NO. 7, and
   reverse 5'-CAATGCCAGCCCCAGCGTCA -3' SEQ ID NO. 8, and
- human β-Actin
   forward: 5'-CCTCTCCCAAGTCCACACAG-3' SEQ ID NO. 9, and
   reverse 5'- CTGCCTCCACCCACTC-3' SEQ ID NO. 10,
   (see Gentschev *et al.,* 2010).

The PCR reaction was run in a T-Gradient Thermoblock PCR machine (Biometra, Göttingen, Germany) under the following conditions: 98°C/1 min, (30 cycles of 98 °C/15 sec, 56°C/15 sec, 72°C/5 sec) and 72°C/5 min.

### 1.4 si-RNA Transfection

For AVEN gene knockdown, a si-RNA-27 kit (OriGene, Rockville, USA) containing three unique human AVEN-27mer si-RNA duplexes (Locus ID 57099) and a Trilencer-27 universal scrambled negative control si-RNA duplex in combination with SiLentFect^{tm} Lipid Reagent (Bio-Rad Laboratories GmbH, München, Deutschland) were used.

Si-RNA sequences (si-AVEN) were as follows:
SEQ ID NO. 1:
   SR311345A rGrCrCrArGrUrGrUrUrUrArCrArGrCrArUrArGrCrArGrATG SEQ ID NO. 2:
   SR311345B rUrGrUrGrGrArUrArGrUrGrArGrUrUrArUrUrGrGrUrUrCGA SEQ ID NO. 3:
   SR311345C rGrCrUrGrCrUrGrArUrUrArArGrArGrArArUrArGrArUrGCA

Negative control sequence (si-control) was as follows:
SEQ ID NO. 4:
   SR30004 rCrGrUrUrArArUrCrGrCrGrUrArUrArArUrArCrGrCrGrUAT

Wherein "r" denotes the sequence being RNA and not DNA.

For si-RNA transfection of the AVEN gene, cells were cultured for 24 h in wells of a 6-well plate to 50-60% confluence. For transfection, SiLentFect™ Lipid Reagent (5 µl/well) and si-RNA (15 µM/well scrambled control or 3x5 µM/well of specific AVEN si-RNA duplexes) were mixed in 100 µl serum-free medium and incubated at room temperature for 20 min. As a control, a sample with SiLentFect^{tm} Lipid Reagent but without si-RNA duplex was used. Cells were incubated for 4 h with 1 ml of serum-free medium and the SiLentFect^{tm}/si-RNA (si-control/si-AVEN) mixture. Afterwards 1 ml serum-containing medium was added and cells were cultivated for 48 or 72 h (final concentration of the si-RNA 37.5 nM). Transfected cells were used for RT-PCR, Western Blot experiments, FACS analysis or cells were infected with VACV.

### 1.5 Western Blot Analysis

For Western Blot analysis, cells were harvested and lysed in RIPA buffer (50 mM Tris-HCl pH 7.4, 150 mM NaCl, 1 mM EDTA, 1 % Triton X-100, 1 % Sodium desoxycholate, 0.1 % SDS, 1 tablet Complete (Roche Diagnostics)). The lysates were separated on a 12 or 14% SDS-PAGE, and afterwards proteins were transferred onto a nitrocellulose transfer membrane (Whatman GmbH, Dassel, Germany).

Polyclonal rabbit antibodies against AVEN (#2300, 1:100, Cell Signaling Technology, Inc., Danvers, USA), caspase-3 (sc-7148, 1:100, Santa Cruz Biotechnology Inc., Heidelberg, Germany) caspase-9 (#9502, 1:100, Cell Signaling technology, Inc.) and GAPDH (ab 8245, 1:2500, abcam, Cambridge, UK) were used and incubated O/N at 4°C. The secondary antibody (horseradish peroxidase-conjugated anti-rabbit IgG; Santa Cruz Biotechnology Inc.) exposure was for 1 h at a 1:5000 dilution in PBS-Tween. Actin was detected using monoclonal anti-mouse actin antibody (ab6276, 1:10,000, abcam) and a secondary horseradish peroxidase-conjugated antibody (anti-mouse IgG, ab6728, 1:2000, abcam). Peroxidase-bound protein bands were visualized using the enhanced chemiluminescence method.

### 1.6 Flow Cytometry

Cells transfected with si-RNA AVEN (si-AVEN) or scrambled RNA (si-control)

| si-AVEN | SEQ ID NO. 1, 2 and 3: |
|---|---|
| SR311345A | rGrCrCrArGrUrGrUrUrUrArCrArGrCrArUrArGrCrArGrATG |
| SR311345B | rUrGrUrGrGrArUrArGrUrGrArGrUrUrArUrUrGrGrUrUrCGA |
| SR311345C | rGrCrUrGrCrUrGrArUrUrArArGrArGrArArUrArGrArUrGCA |
| | |
| si-control | SEQ ID NO. 4: |
| SR30004 | rCrGrUrUrArArUrCrGrCrGrUrArUrArArUrArCrGrCrGrUAT |

were cultured in wells of a 6-well plate for 48 or 72 h and analyzed by FACS analysis prior to viral infection as well as 24 or 48 hours post infection (hpi). Cells were trypsinized with 300 µl trypsin/EDTA (PAA Laboratories) until all cells were detached. The reaction was stopped by adding 600 µl of culture medium. Samples were centrifuged at 2000 rpm for 3 min, 4 °C.

For cell cycle analysis and detection of DNA fragmentation (sub-G1 phase) (Krysko *et al.,* 2008; Darzynkiewicz *et al.,* 1997) cell pellets were resuspended in a solution of 100 µl PBS + 2% FBS and 5 µl propidium iodide (PI, Sigma-Aldrich). Prior to measurement, the suspension was frozen in liquid nitrogen and thawed at 37°C.

To distinguish apoptotic from necrotic cells, a FITC Annexin V Apoptosis Detection Kit with 7-AAD (BioLegend Inc. San Diego, USA) was used. For this, cells were washed once with PBS, resuspended in 100 µl Annexin V Binding Buffer supplemented with 2 µl FITC-Annexin V and 2 µl of 7-AAD cell viability staining solution. In addition, the combination of Annexin V-APC (Immunotools GmbH, Oldenburg, Germany) and PI was used. Cells were analyzed, using an Accuri C6 Cytometer and FACS analysis software CFlow Version 1.0.227.4 (Accuri Cytometers, Inc. Ann Arbor, MI USA). Cell debris was excluded from the measurements by defining a proper gate for the cell population of interest by using forward (FSC) and sideward (SSC) scatter. 10 000 events were analyzed. Experiments were performed in triplicate and repeated twice.

### 1.7 Replication analysis and standard viral plaque assay

Cells transfected with si-RNA AVEN (si-AVEN) and scrambled RNA (si-control) were cultured in wells of a 6-well plate for 48 or 72 h and were infected with GLV-1h68 at a multiplicity of infection (MOI) of 0.1, 0.5 or 1.0 in 1 ml infection medium with 2% FBS for 1 h. Every 15 min, cells were agitated slightly for homogenous infection. Infection medium was collected and replaced by 1.5 ml culture medium. Cells were resuspended in 1 ml PBS after harvesting and supernatants were collected separately 24 and 48 hours post infection (hpi). Prior to analysis three freeze and thaw cycles in liquid nitrogen were accomplished to release viral particles. All samples were infected in triplicate. Serial dilutions of the samples were titrated on 100% confluent CV-1 monolayers by standard viral plaque assay in duplicate.

### 1.8 MTT-Assay

Cells were infected with GLV-1h68 at an MOI of 0.1 and 1.0. Cell viability was determined 24, 48 and 72 h after viral infection. For this purpose, culture medium was replaced by 500 µl sterile filtrated MTT 3-(4,5-Dimethylthiazol-2-yl)-2,5-diphenyltetrazolium Bromide (2.5 mg/ml; Sigma) dissolved in RPMI-1640 without Phenol Red (Sigma-Aldrich) and incubated for 2 h at 37°C. The color reaction was stopped by adding 1 N HCl diluted in isopropyl alcohol (Sigma-Aldrich). The optical density was measured at a wavelength of 570 nm in an Elisa Photometer Sunrise^{tm} (TECAN Group, Männedorf, Germany). Uninfected cells were used as positive control, defined as 100% viable. The experiment was performed twice in triplicate.

### 1.9 Statistical analysis

To determine significance between 2 conditions or groups, a 2-tailed t-test with unequal variances was used (Excel 2010 for Windows). P-values were defined as follows *p<0.05, **p<0.01, ***p<0.001.

### 1.10 Gene expression data

Gene expression data was generated as described previously (Reinboth *et al.,* 2012). In brief, 1936-MEL, 888-MEL, GI-101A, and HT-29 cells were infected with GLV-1h68 at an MOI of 0.01 or mock-treated with infection medium (2%-FBS). Cells were harvested at respective time points and total RNA isolated via Qiagen miRNeasy mini kit. RNA was amplified (Wang *et al.,* 2000) and purified for microarray performance (36k human array platforms, manufactured in-house at the Department of Transfusion Medicine, NIH, Bethesda, MD, USA). Combined peripheral blood mononuclear cells (PBMCs) from 4 normal donors were utilized as a reference. Reference and sample aRNA were labeled directly with Cy3 and Cy5, respectively (two-color system) and cohybridized to the array platform. Following incubation, washing steps, and scanning (Agilent scanner) gene expression data was uploaded to the mAdb databank (http://nciarray.nci.nih.gov). Data from human in-house arrays was processed via BRBArrayTools (Simon *et al.,* 2007) including a quality-control for imperfect spots (Spot filter), normalization via median log-ratio subtraction, and specification of the maximum intensity ratio plus the truncation of larger intensities ratios to this maximum.

### 1.11 Dynamic modeling

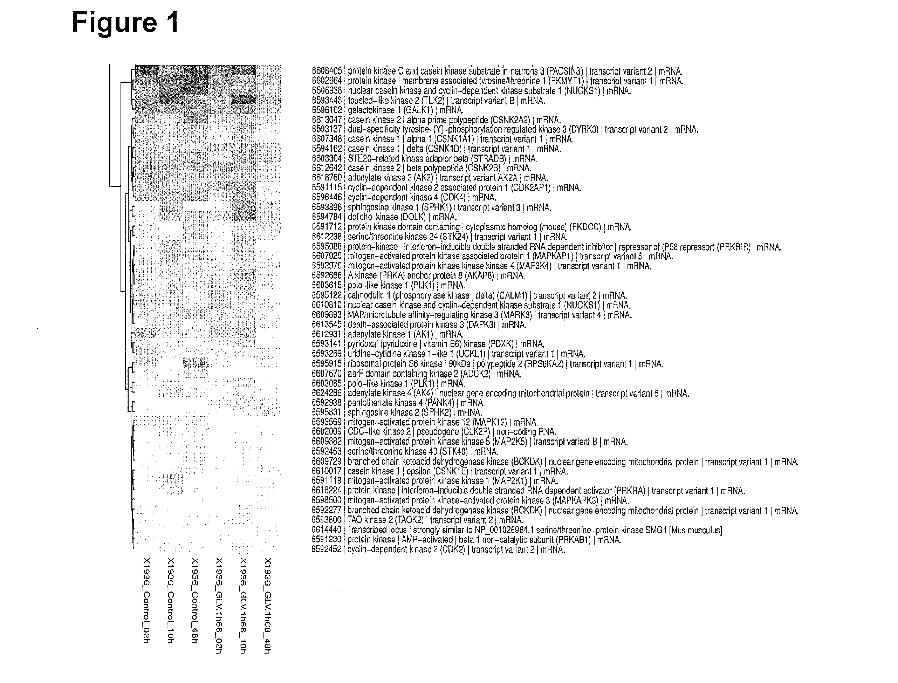

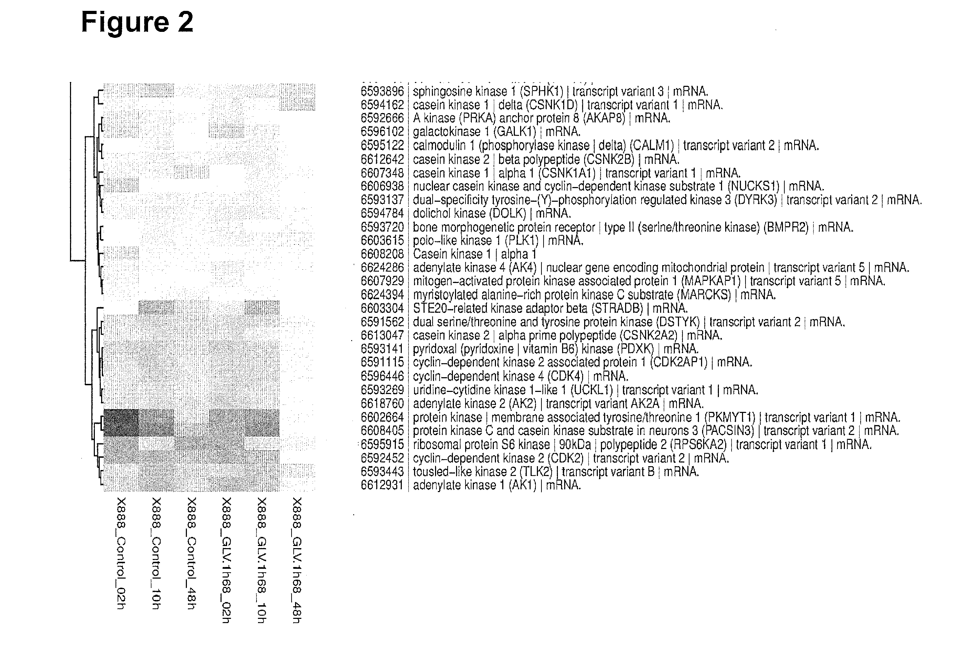

Gene expression data were obtained for 1936-MEL, 888-MEL, GI-101A, and HT-29 cell lines. For the 18,800 probes of the 1936-MEL and 888-MEL cell lines and the 32,000 probes of the GI-101A and HT-29 cell lines, a gene expression library was set up in MySQL. Identification of the most relevant genes from the general apoptosis network was done in R (R Core Team, 2013) and custom scripting for the "pheatmap"- package (Kolde, 2013). The dataset of interest consisted primarily of caspases, protein kinases and casein kinases (Figures 1 to 3). The dynamic modeling was done with CellDesigner 3.5.2 (Funahashi *et al.,* 2008) and consisted of the apoptosis, MAPK, p53, WNT, Hedgehog and mitochondrial Ca²⁺- signaling. The evaluation of the resulting signal strength was done by SQUAD (Di Cara *et al.,* 2007).

### 2. Results

To elucidate genetic differences and the specific role and function of the anti-apoptotic factor AVEN for oncolytic vaccinia virus (VACV) therapy, we chose a panel of four human tumor cell lines of different histological background for our study, namely:
888-Mel,
1936-MEL,
GI-101A, and
HT-29.

The various aspects of oncolytic VACV therapy have already been studied in detail in these cell lines (see Ehrig *et al.,* 2013; Zhang *et al.,* 2007; Worschech *et al.,* 2009; Reinbooth *et al.,* 2012; Ascertio *et al.,* 2011; Weibel *et al.,* 2013-b; Wang *et al.,* 2012; Chen *et al.,* 2011; Weibel *et al.,* 2011).

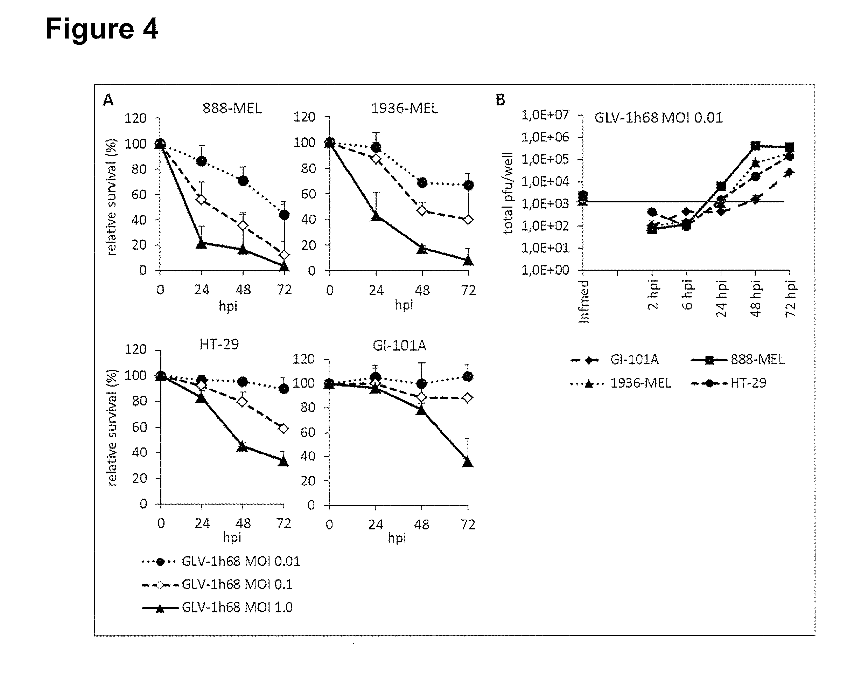

We performed an *in vitro* MTT-cell survival assay to compare GLV-1h68-induced cell death in 888-MEL, 1936-MEL, GI-101A and HT-29 (Figure 4A). A time course from 24-72 h and three different MOIs (0.01, 0.1 and 1.0) were analyzed. Virus-induced cell death was more pronounced in 888-MEL and 1936-MEL than in HT-29 and GI-101A at all MOIs tested. By standard viral plaque assay (MOI 0.01) we could show that all four cell lines were permissive for viral replication, indicated by an increase of the virus titer (pfu/ml) in cells and supernatants from the initial infection medium (Figure 4B). In accordance with the MTT-cell survival assay strongest viral replication was detected in 888-MEL with a virus titer (total pfu/well) higher than the infection medium already 24 hpi. Weakest viral replication was detected in GI-101A where active viral replication started for the first time at 72 hpi. Taken together, the infection/replication pattern to GLV-1h68 is different in all four cell lines analyzed and does not correspond to the *in vivo* T.I. classification of responder (888-MEL and GI-101A) and non-responder (1936-MEL and HT-29) cell lines (Worschech et al., 2009).

We could show that 1936-MEL and 888-MEL show similar gene expression patterns (see Figures 1 to 2). The expression of genes coding for kinases in general - and protein kinases and casein kinases in particular - are elevated in both, mock- and with GLV-1h68 infected cells. The proteins encoded by these genes are located in the WNT, Hedgehog, MAPK and apoptosis pathways. Of special interest are the casein kinases. These proteins have a very significant role to regulate Glycogen synthase kinase 3 beta (GSK3β), the primary inhibitor of β-Catenin. β-Catenin has been reported previously to be responsible for tumor proliferation, e.g. in basal cell carcinoma (Saldanha *et al.,* 2004) or in prostate cancer (Kypta and Waxman, 2012). According to literature up-regulated casein kinases lead to the following changes of the β-Catenin activity:
1. GSK3β is inhibited by a CK1ε / CK2 / DVL scaffold in the WNT- signaling pathway. GSK3β itself is responsible for an inhibition of β-Catenin. Therefore the inhibition of GSK3β leads to higher β-Catenin activity.
2. β-Catenin itself can be activated by another casein kinase: CK1α. CK1α is up-regulated in its gene expression in our data.

Although the up-regulation of the casein kinase genes in the melanoma cell line 888-MEL is not as high as in the melanoma cell line 1936-MEL, it is still significant. Modeling of these differences in gene expression on an apoptosis signaling network, showed to specific differences in tumor survival and death: The *in silico* modeling in CellDesigner and SQUAD shows a twice as high survival rate of the 1936-MEL than observed for 888-MEL when compared to an *in silico* untreated mock cancer cell line, whereas the rate of cell death is higher in 888-MEL as in 1936-MEL (Figure 5). Therefore these differences in gene expression are sufficient to render the oncolytic virus GLV-1h68 against 1936-MEL almost ineffective, whereas it is very effective against 888-MEL.

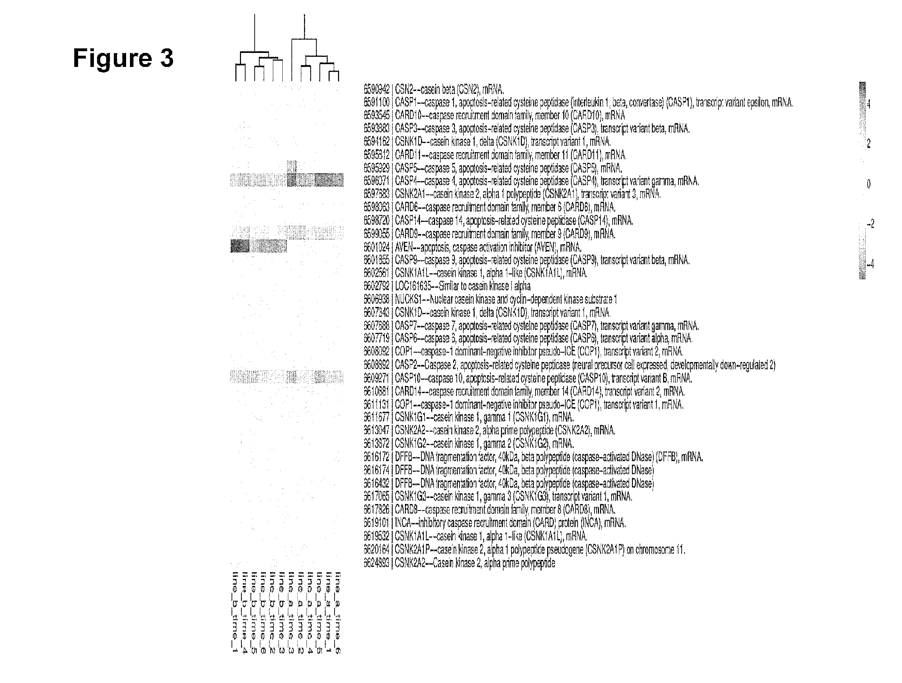

The colorectal adenocarcinoma HT29 and the breast cancer cell line GI-101A show a different gene expression than the MEL cell lines: Here mainly caspases in the apoptosis pathway are up-regulated (Figure 3). In the case of HT-29 the gene *AVEN,* which is only upregulated in this cell line out of the four studied here, is of particular interest: it works as a caspase-9 inhibitor and anti-apoptotic BCL2-family activator (Chau *et al.,* 2000).

*In silico* modeling of all four cell lines shows a significant increase in tumor survival when compared to an untreated GI-101A cell line. The rate of survival is even higher than in 1936-MEL and leads to the conclusion that AVEN plays a very vital role in resistance against the oncolytic effects of the GLV-1h68 virus. The *in silico* findings are conform to previous *in vitro* findings (Reinboth *et al.,* 2012) of the efficacy of GLV-1h68 against 1936-MEL, 888-MEL, GI-101A and HT-29.

To verify the *in silico* modeling predictions, further cell culture studies were next performed: AVEN is found ubiquitously expressed in various cell lines and tissues (Chau *et al.,* 2000). We wanted to verify this for 888-MEL, 1936-MEL, GI-101A and HT-29. Cells were cultured for 24 and 48 h and AVEN gene expression was tested on RNA level (Figure 6A). AVEN could be detected in all four cell lines cultured for 24 and 48 h respectively. GAPDH, a housekeeping gene, was used as control. Calculation of the ratio between AVEN and GAPDH gene expression levels revealed a higher AVEN gene expression after 24 (ratio >1) than after 48 h (ratio <1) in culture (Figure 6B). This could be due to increasing cell densities. The AVEN/GAPDH ratio was highest in HT-29 compared to the other cell lines. Testing AVEN on protein level exemplarily shown for HT-29 and 1936-MEL revealed that it was expressed in both cell lines 6, 24 and 48 hpi in control and GLV-1h68 (MOI 1.0) infected samples (Figure 6C).

As AVEN was found to be a novel regulator of the apoptosome complex and further was identified as potential biomarker e.g. in hematological malignancies (Chau *et al.,* 2000; Eißmann et al., 2012), we wanted to elucidate whether the anti-apoptotic factor AVEN would affect the permissiveness of the weak/non-responder cell line HT-29 to GLV-1h68 infection. RNA interference was used to down-regulate AVEN in HT-29 cells. For this, cells were transfected for 48 to 72 h with a si-RNA targeting AVEN (si-AVEN) and a scrambled RNA (si-control) as control. AVEN gene expression was significantly down-regulated in HT-29 cells compared to cells transfected with si-control (Figure 7A).

To verify whether the knockdown of AVEN would enhance apoptotic characteristics in the cancer cells, DNA fragmentation as one characteristic to distinguish apoptotic from necrotic cell death (Gentschev *et al.,* 2010) was tested. Down-regulation of the anti-apoptotic factor AVEN in HT-29 cells revealed a significant increase of DNA-fragmentation, detected as an increase of cells in sub-G1 phase in si-AVEN transfected cells compared to si-control transfected cells (Figure 7B). No significant difference could be observed in Annexin⁺/7AAD⁺/⁻ (apoptotic) or Annexin⁻ /7AAD⁺ (necrotic) cells between si-control and si-AVEN transfected HT-29 cells (Figure 7C). Western Blot analysis revealed the expression of procaspase-3 (32 kDa in both si-AVEN and si-control transfected cells. The cleaved and activated form of the effector caspase-3 was not expressed (Figure 7D). In accordance with the literature, down-regulation of AVEN results in an activation and cleavage of the initiator caspase-9 (35 kd) (Figure 7D). GAPDH was used as loading control.

Following the functional proof of AVEN down-regulation in HT-29 cells, we tested GLV-1h68 replication (MOI 1.0) and the infection rate (GFP⁺ cells) in these cells. A significant reduction of the virus titer (Figure 7E) and a significantly smaller number of GFP⁺ cells (Figure 7F) were detected in si-AVEN transfected cells compared to si-control transfected cells. Percentage of cells in sub-G1 phase 24 hpi was significantly enhanced in GLV-1h68 infected si-AVEN transfected HT-29 cells compared to si-control transfected HT-29 cells (Figure 7G).

Taken together, a reduced infection rate (GFP⁺ cells) and an impaired viral replication took place after down-regulation of the anti-apoptotic factor AVEN in HT-29 cells. However, this down-regulation also resulted in an increase of apoptosis in HT-29.

To see, whether the observed VACV effects are a general phenomenon or unique for the non-responder cell line HT-29, knockdown of AVEN was also tested in the responder cell line 888-MEL and weak responder 1936-MEL (Figures 8 and 9). In these analyses similar results could be obtained: viral replication is reduced but at the same time apoptosis of cancer strains is enhanced.

The features disclosed in the foregoing description, in the claims and/or in the accompanying drawings may, both separately and in any combination thereof, be material for realizing the invention in diverse forms thereof.

### REFERENCES

Advani, S. J. et al. Preferential replication of systemically delivered oncolytic vaccinia virus in focally irradiated glioma xenografts. Clin. Cancer Res. 18, 2579-90 (2012).
Ascierto, M. L. et al. Permissivity of the NCI-60 cancer cell lines to oncolytic Vaccinia Virus GLV-1h68. BMC Cancer 11,451 (2011).
Bartlett DL, Liu Z, Sathaiah M, Ravindranathan R, Guo Z, He Y, Guo ZS. Oncolytic viruses as therapeutic cancer vaccines., Mol. Cancer, vol. 12, no. 1, p. 103, Jan. 2013.
Bao Q S. Y. Apoptosome: a platform for the activation of initiator caspases, Cell Death Differ., vol. 14, pp. 56-65, 2007.
Bourke, M. G. et al. The emerging role of viruses in the treatment of solid tumours. Cancer Treat. Rev. 37, 618-32 (2011).
Bratton, S. B. & Salvesen, G. S. Regulation of the Apaf-1-caspase-9 apoptosome J. Cell Sci. 123, 3209-14 (2010).
Brenner, D. & Mak, T. W. Mitochondrial cell death effectors. Curr. Opin. Cell Biol. 21, 871-7 (2009).
Cecil A, Gentschev I, Adelfinger M, Nolte I, Dandekar T, Szalay AA. Antigen profiling analysis of vaccinia virus injected canine tumors: Oncolytic virus efficiency predicted by boolean models, Bioengineered, vol. 5, no. 5, pp. 1-7, Sep. 2014.
Chau, BN, Cheng, EH, Kerr, DA, Hardwick, JM. Aven, a novel inhibitor of caspase activation, binds Bcl-xL and Apaf-1. Mol. Cell 6, 31-40 (2000).
Chen, NG, Yu, YA, Zhang, Q, Szalay, AA. Replication efficiency of oncolytic vaccinia virus in cell cultures prognosticates the virulence and antitumor efficacy in mice. J. Transl. Med. 9, 164 (2011).
Chen NG, Szalay AA. "Oncolytic Virotherapy of Cancer" in Cancer Management in Man: Chemotherapy, Biological Therapy, Hyperthermia and Supporting Measures. 524 (Springer Netherlands, 2011), paged 295-316. doi:10.1007/978-90-481-9704-0
Choi, J. et al. Aven overexpression: association with poor prognosis in childhood acute lymphoblastic leukemia. Leuk. Res. 30, 1019-25 (2006).
Darzynkiewicz Z, Juan G, Li X, Gorczyca W, Murakami T, T. F. Cytometry in cell necrobiology: analysis of apoptosis and accidental cell death (necrosis). Cytometry 27, 1-20 (1997).
Di Cara, A., Garg, A., De Micheli, G., Xenarios, I. & Mendoza, L. Dynamic simulation of regulatory networks using SQUAD. BMC Bioinformatics 8, 462 (2007).
Donat, U. et al. Characterization of Metastasis Formation and Virotherapy in the Human C33A Cervical Cancer Model. PLoS One 9, e98533 (2014).
Donat, U. et al. Preferential colonization of metastases by oncolytic vaccinia virus strain GLV-1h68 in a human PC-3 prostate cancer model in nude mice. PLoS One 7, e45942 (2012).
Ehrig, K. et al. Growth inhibition of different human colorectal cancer xenografts after a single intravenous injection of oncolytic vaccinia virus GLV-1h68. J. Transl. Med. 11, 1 (2013).
Eißmann, M. et al. Overexpression of the anti-apoptotic protein AVEN contributes to increased malignancy in hematopoietic neoplasms. Oncogene 32, 2586-2591 (2012).
Figueroa B Jr, Chen S, Oyler GA, Hardwick JM, Betenbaugh MJ. Aven and Bcl-xL enhance protection against apoptosis for mammalian cells exposed to various culture conditions. Biotechnol Bioeng. 2004;85(6):589-600.
Funahashi A, Matsuoka Y, Jouraku A, Morohashi M, Kikuchi N, K. H. CellDesigner 3.5: A Versatile Modeling Tool for Biochemical Networks. Proc. IEEE 96, 1254-1265 (2008).
Gentschev I, Donat U, Hofmann E, Weibel S, Adelfinger M, Raab V, Heisig M, Chen N, Yu YA, Stritzker J, Szalay AA. Regression of human prostate tumors and metastases in nude mice following treatment with the recombinant oncolytic vaccinia virus GLV-1h68. J. Biomed. Biotechnol. 2010, 489759 (2010).
Goldufsky J, Sivendran S, Harcharik S, Pan M, Bernardo S, Stern RH, Friedlander P, Ruby CE, Saenger Y, Kaufman HL. Oncolytic virus therapy for cancer. Oncolytic Virotherapy 2013, 2:31-46. Review.
Guo, J. Y. et al. Aven-dependent activation of ATM following DNA damage. Curr. Biol. 18, 933-42 (2008).
Hajra KM, L. J. Apoptosome dysfunction in human cancer. Apoptosis 9, 691-704 (2004).
Hanahan, D., Weinberg, R. A. & Francisco, S. The Hallmarks of Cancer Review. Cell 100, 57-70 (2000).
Hanahan, D. & Weinberg, R. A. Hallmarks of cancer: the next generation. Cell 144, 646-74 (2011).
Hawley RG, Chen Y, Riz I, Z. C. An Integrated Bioinformatics and Computational Biology Approach Identifies New BH3-Only Protein Candidates. Open Biol J5, 6-16 (2012).
Kolde, R. pheatmap: Pretty Heatmaps. (2013). at <ttp://cran.r-project.org/web/packages/pheatmap/index.html>
Kotliarova, S. et al. Glycogen synthase kinase-3 inhibition induces glioma cell death through c-MYC, nuclear factor-kappaB, and glucose regulation. Cancer Res. 68, 6643-51 (2008).
Krysko, D. V, Vanden Berghe, T., D'Herde, K. & Vandenabeele, P. Apoptosis and necrosis: detection, discrimination and phagocytosis. Methods 44, 205-21 (2008).
Kutuk, O., Temel, S. G., Tolunay, S. & Basaga, H. Aven blocks DNA damage-induced apoptosis by stabilising Bcl-xL. Eur. J Cancer 46, 2494-505 (2010).
Kypta RM, Waxman J. Wnt/β-catenin signalling in prostate cancer. Nat Rev Urol. 2012;(8):418-28.
Ledgerwood, E. C. & Morison, I. M. Targeting the apoptosome for cancer therapy. Clin. Cancer Res. 15, 420-4 (2009).
Liu, T., Galanis, E. & Kim, D. Clinical trial results with oncolytic virotherapy: a century of promise, a decade of progress. Nat. Clin. Pract. Oncol. 4, 101-117 (2007).
Melzer IM, Fernández SB, Bösser S, Lohrig K, Lewandrowski U, Wolters D, Kehrloesser S, Brezniceanu ML, Theos AC, Irusta PM, Impens F, Gevaert K, Zörnig M. The Apaf-1-binding protein Aven is cleaved by Cathepsin D to unleash its anti-apoptotic potential. Cell Death Differ. 2012;19(9):1435-45.
Patil SS, Gentschev I, Adelfinger M, Donat U, Hess M, Weibel S, Nolte I, Frentzen A, Szalay AA. Virotherapy of canine tumors with oncolytic vaccinia virus GLV-1h109 expressing an anti-VEGF single-chain antibody., PLoS One, vol. 7, no. 10, p. e47472, Jan. 2012.
Paydas, S. Survivin and aven: two distinct antiapoptotic signals in acute leukemias. Ann. Oncol. 14, 1045-1050 (2003).
Reinboth, J. et al. Correlates between host and viral transcriptional program associated with different oncolytic vaccinia virus isolates. Hum. Gene Ther. Methods 23, 285-96 (2012).
Saldanha, G., Ghura, V., Potter, L. & Fletcher, A. Nuclear beta-catenin in basal cell carcinoma correlates with increased proliferation. Br. J. Dermatol. 151, 157-64 (2004).
Simon, R., Lam, A., Li, M. & Ngan, M. Analysis of gene expression data using BRB-array tools. Cancer Inform. 2007; 3: 11-17.
Team, R. core. R: A Language and Environment for Statistical Computing. (2013). at http://www.r-project.org
Wang, E., Miller, L. & Ohrnnacht, G. High-fidelity mRNA amplification for gene profiling. Natur biotech 18, 457-459 (2000).
Wang, H., Chen, N. G., Minev, B. R. & Szalay, A. A. Oncolytic vaccinia virus GLV-1h68 strain shows enhanced replication in human breast cancer stem-like cells in comparison to breast cancer cells. J. Transl. Med. 10, 167 (2012).
Weibel, S. et al. Viral-mediated oncolysis is the most critical factor in the late-phase of the tumor regression process upon vaccinia virus infection. BMC Cancer 11, 68 (2011).
Weibel, S. et al. Treatment of malignant effusion by oncolytic virotherapy in an experimental subcutaneous xenograft model of lung cancer. J. Transl. Med. 11, 106 (2013-a).
Weibel, S. et al. Imaging of intratumoral inflammation during oncolytic virotherapy of tumors by 19F-magnetic resonance imaging (MRI). PLoS One 8, e56317 (2013-b).
Worschech, A. et al. Systemic treatment of xenografts with vaccinia virus GLV-1h68 reveals the immunologic facet of oncolytic therapy. BMC Genomics 10, 301 (2009).
Zhang YA, Nemunaitis J, Samuel SK, Chen P, Shen Y, Tong AW. Antitumor activity of an oncolytic adenovirus-delivered oncogene small interfering RNA. Cancer Res. 2006; 66(19):9736-43.
Zhang Q, Yu YA, Wang E, Chen N, Danner RL, Munson PJ, Marincola FM, Szalay AA. Eradication of Solid Human Breast Tumors in Nude Mice with an Intravenously Injected Light-Emitting Oncolytic Vaccinia Virus. Cancer Res. 67, 10038-10046 (2007).

## Claims

1. A recombinant virus comprising in its viral genome the genetic information for inhibiting the gene AVEN in a target or host cell,
wherein said genetic information is selected from genetic information for a si-RNA, sh-RNA, antisense-DNA, antisense-RNA, sc-AB or sd-AB.

2. The recombinant virus of claim 1, wherein the genetic information for said AVEN si-RNA, sh-RNA, antisense DNA, antisense-RNA, sc-AB or sd-AB is combined with / linked to a late/very late promoter,
wherein said very late promoter is preferably selected from PE/L (early/late promoter) or P11 (late/very late promoter).

3. The recombinant virus of claim 1 or 2, wherein the recombinant virus is selected from:
- the *Poxviridae, Adenoviridae, Reoviridae* and *Herpesviridae* families of dsDNA viruses,
- the *Picornaviridae, Rhabdoviridae, Paramyxoviridae* and *Togaviridae* families of ssRNA viruses, and
- the *Retroviridae* family of retro-transcribing viruses.

4. The recombinant virus of claim 3, wherein the recombinant virus is
- an attenuated oncolytic virus,
such as
an attenuated adenovirus (e.g. ONYX-105),
an attenuated vaccinia virus (VACV),
an attenuated poliovirus (e.g. PV1),
- a replication-competent oncolytic virus,
such as
a replication-competent oncolytic adenovirus (e.g. ONYX-411),
a replication-competent oncolytic vaccinia virus (VACV),
a replication-competent oncolytic seneca valley virus (e.g. SVV-01),
a replication-competent oncolytic reovirus.

5. The recombinant virus of claim 3 or 4, which comprises or carries
- in its viral genome deletional mutation(s) in nonessential gene(s) and/or in small ORF(s),
- foreign or heterologous genes,
such as
- genes encoding for tumor suppressor proteins, immunostimulatory proteins and cytokines,
- genes encoding for pro-drug activating enzymes,
- genes encoding for marker proteins,
such as fluorescent proteins, e.g. GFP or its derivatives,
- genes encoding for glucoronidase as a marker for oncolysis.

6. The recombinant virus of claim 5, which is an oncolytic vaccinia virus (VACV) which comprises or carries in its viral genome deletional mutation(s)
- in nonessential gene(s),
such as *JR2* (encoding thymidine kinase, TK), *A56R* (encoding hemagglutinin), vaccinina growth factor gene,
and/or
- in small ORF(s)
such as *F14.5,*
and comprises or carries foreign or heterologous genes,
such as renilla luciferase-aequorea GFP (Ruc-GFP), and/or glucoronidase, and/or transferrin receptor (TFR) and *lacZ,*
which is preferably the oncolytic vaccinia virus strain GLV-1h68

7. The recombinant virus of any of the preceding claims, wherein the si-RNA AVEN comprises or consists of the nucleic acid sequence of SEQ ID NO. 1, 2 or 3.

8. The recombinant virus of any of the preceding claims, wherein the target or host cell is a cancerous or tumor cell.

9. A pharmaceutical composition, comprising
(i) the recombinant virus of any one of claims 1 to 8; and
(ii) optionally, pharmaceutically acceptable carrier(s) and/or excipient(s).

10. The pharmaceutical composition of claim 9, comprising further drug(s), such as chemotherapeutic agent(s), radiotherapeutic agent(s) and/or agent(s) for near infrared laser-induced targeted cancer therapy,
and/or formulated for systemic delivery, tumor injection, intravenous administration,
and/or for intradermal, subcutaneous, intramuscular, intravenous, intraosseous, intraperitoneal, intrathecal, epidural, intracardiac, intraarticular, intracavernous, intracerebral, intracerebroventricular and intravitreal injection(s).

11. Use of the recombinant virus of any of claims 1 to 8 or the pharmaceutical composition of claim 9 or 10, as gene delivery tool and/or for tumor detection.

12. The recombinant virus of any of claims 1 to 8 or the pharmaceutical composition of claim 9 or 10, for use in medicine.

13. The recombinant virus of any of claims 1 to 8 or the pharmaceutical composition of claim 9 or 10, for use in the diagnosis, prevention and/or treatment of a disease,
wherein said disease is cancer.

14. The recombinant virus or pharmaceutical composition for use according to claim 13, for use in oncolytic virotherapy.

15. The recombinant virus or pharmaceutical composition for use according to claim 13 or 14, for use in combination with other therapies, preferably other cancer therapies, such as chemotherapy and/or radiation therapy,
and/or wherein administration is systemic, intravenous, via injection into tumor,
and/or via intradermal, subcutaneous, intramuscular, intravenous, intraosseous, intraperitoneal, intrathecal, epidural, intracardiac, intraarticular, intracavernous, intracerebral, intracerebroventricular and intravitreal injection(s).
